# EUROPÄISCHE PATENTSCHRIFT

(11) **EP 1 243 231 B1**
(45) Veröffentlichungstag und Bekanntmachung des Hinweises auf die Patenterteilung: **18.03.2009**
(21) Anmeldenummer: 02001493.2
(22) Anmeldetag: 22.01.2002
(51) Int. Cl.: A61C 13/00, A61K 6/083

(54) **Verfahren zur Herstellung von dentalen Formteilen**
Method for producing dental workpieces
Procédé de fabrication de pièces dentaires

(30) Priorität: 23.03.2001 DE 10114290
(43) Veröffentlichungstag der Anmeldung: 25.09.2002
(73) Patentinhaber: Ivoclar Vivadent AG, 9494 Schaan (LI)
(72) Erfinder: Moszner, Prof. Dr. Norbert, FL-9492 Eschen (LI); Burgath, Dr. Armin, D-78351 Bodmann-Ludwigshafen (DE); Mülhaupt, Prof. Dr. Rolf, D-79117 Freiburg (DE); Salz, Dr. Ulrich, D-88131 Lindau (DE); Rheinberger, Dr. Volker, FL-9490 Vaduz (LI); Landers, Rüdiger, D-79104 Freiburg (DE)
(74) Vertreter: Baronetzky, Klaus

(56) Entgegenhaltungen:
- WO-A-01/13814
- WO-A-01/15620
- DE-A- 4 408 754
- DE-A- 19 938 463
- US-A- 5 204 124
- US-A- 5 690 490
- US-A- 5 869 170

## Beschreibung

Die Erfindung betrifft die Verwendung des 3D-Plottings zur Herstellung von Dentalprodukten unter Verwendung von schmelzbaren, kondensierbaren, thermisch oder mit UV- bzw. sichtbarem Licht härtbaren ungefüllten oder gefüllten Materialien mittels eines computergesteuerten 3D-Plotters.

Für die Herstellung von Zahnersatz oder Teilen einer Zahnrestauration, wie Inlays, Onlays, Brücken, Kronen oder Prothesen werden heute noch überwiegend die traditionellen mehrstufigen Abform- und Giessverfahren eingesetzt. Danach erfolgt in weiteren Schritten die Herstellung der einzelnen dentale Formteile auf übliche Weise. Diese Verfahren sind erprobt und bewährt, jedoch mit einem hohen Fertigungsaufwand verbunden. Daher wurden insbesondere in den letzten Jahren eine Reihe von Verfahren entwickelt, um den Aufwand zu reduzieren und die Qualität zu verbessern.

In den letzten Jahren, insbesondere seit 1987, als es erstmals gelang, dreidimensionale Modelle in einem Arbeitsgang direkt auf der Grundlage von Computerdaten herzustellen, hat sich der Begriff "Rapid Prototyping" in der Umgangssprache als ein Synonym für Verfahren eingebürgert, mit dessen Hilfe es möglich wurde, Computerdaten mit verschiedenen Geräten oder Anlagen in Modelle mit verschiedenster Qualität und Haltbarkeit herzustellen. Bei der formfreien Fertigung werden dreidimensionale Computerbilder in Schichten zerlegt, die im computerunterstützten Fertigungsverfahren schichtweise zum realen dreidimensionalen Objekt zusammengefügt werden. Während sich die ersten Modelle überwiegend um Anschauungsmodelle ohne eigentlichen Gebrauchswert handelte, werden mit diesen Verfahren heute bereits Funktionsmodelle hergestellt. Auch Kleinserien lassen sich mit diesen Verfahren preisgünstig herstellen. Während in den ersten Jahren der technisch faszinierenden aber wirtschaftlich unattraktiven Modellbauverfahren vorwiegend lichthärtende Acrylharze (Stereolithographie) verwendet wurden, hat sich in den Jahren die Palette der einzusetzenden Materialien deutlich vergrössert. So wurden die verschiedenen Verfahren des "Rapid Prototyping" auch für die Herstellung von Dentalprodukten eingesetzt. Dies sind vor allem das selektive Lasersintern, das 3D-Printing oder die Stereo-Lithographie.

Die EP-A-1 021 997 beschreibt die Anwendung des Laser-Sinterverfahrens für die Herstellung von Zahnrestaurationen, bei dem aus einem sinterfähigen Pulver schichtweise Formkörper aufgebaut werden, indem jede Schicht nach dem Aufbau schrittweise durch eine Bestrahlung mit einem Laserstrahl gesintert wird. Die Steuerung des Prozesses unterliegt dabei Daten, die die Konfiguration des Formkörpers in der jeweiligen Schicht repräsentieren. Als Werkstoff wird ein Legierungspulver in homogener Zusammensetzung verwendet. Der Nachteil besteht jedoch darin, dass Hohlräume zwischen den Pulvermaterialien nicht auszuschliessen sind. Weiterhin sind nach dieser Druckschrift keine gefüllten Kunststoffe als Pulver einsetzbar.

In der WO 97/29901 wird ein Verfahren (Stereolithographie) und ein Gerät zur Herstellung dreidimensionaler Teile aus einem flüssigen und härtbaren Medium beschrieben. Dabei wird das Teil schichtweise aufgebaut, indem jede einzelne Schicht mit einem Laser abgefahren und dabei ausgehärtet wird. Danach wird mittels eines Abstreifers die nächste Schicht des härtbaren Materials aufgetragen und ebenso gehärtet. Auch hierbei handelt es sich um die Beschreibung eines Modellbauverfahrens, das die Verwendung von Materialien für dentale Anwendungen nicht beschreibt.

Auch aus der DE 199 38 463 A1 ist die Zusammensetzung eines flüssigen lichthärtbaren Kunststoffs sowie ein Verfahren bekannt, bei dem aus diesem Material ein dreidimensionales Formteil erstellt wird. In einem Behälter mit dem härtbaren flüssigen Kunststoff wird auf einer absenkbaren Plattform computergesteuert mittels Laser schichtweise das Formteil erstellt. Die erreichbare Schichtdicke je Durchgang von max ca. 0,25 mm ist dabei auf die Erstellung von Modellen beschränkt.

Aus der WO 95/28688 ist ein stereolithographisches Verfahren zur Herstellung von Dentalimplantaten bekannt. Bei diesem Verfahren wird ein Modell hergestellt und mittels einer CAD-Anwendung modelliert, wobei gemäß der Beschreibungseinleitung bekannt ist, für die Rekonstruktion ein von einem Computertomographen gescanntes Bild zu verwenden, um die relative Lage des Prothesenzahns gegenüber dem darunterliegenden Kieferknochen erfassen zu können und gegebenenfalls Korrekturen vornehmen zu können. Wie bereits dort festgestellt, stellt dies ein zeitaufwendiges Verfahren dar, zudem die Computertomographen vergleichsweise sehr teuer und keineswegs regelmässig in Zahnarztpraxen vorhanden sind.

Es sind weiterhin zahlreiche Verfahren bekannt, bei denen Gegenstände basierend auf Modellen oder Vorlagen schnell hergestellt werden können. Ein Beispiel dafür ist aus der US-A-5.370.692 zu entnehmen. Bei diesem Verfahren (Modifiziertes Selektives Lasersintern), das sich schwerpunktmässig auf das Replizieren von Knochenmaterial richtet, wobei auch dentale Implantate angesprochen sind, wird das Implantat dadurch hergestellt, dass schichtweise Implantatschichten aufgebracht werden, wobei der Schritt des Aufbringens selbst über eine Art Drucken erfolgt. Bevorzugt werden keramische Teilchen oder Polymerteilchen miteinander verbunden, um das Implantat zu bilden. Ein derartiges Druckverfahren ist jedoch nicht für alle Anwendungsfälle geeignet. Zudem lässt sich Keramik in der Regel nicht ohne Schrumpfungsprozesse sintern. Ein weiteres Problem bei der Realisierung von Dental-Prothesen liegt darin, dass stets für die Zahnaufstellung eine Kontrolle im Artikulator vorgenommen werden muss. Dies erfolgt bei der herkömmlichen Technik über die Herstellung einer Wachsprothese als Zwischenschritt. Eine derartige Wachsprothese lässt sich andererseits von einem Modell praktisch nicht zerstörungsfrei entnehmen, nachdem zumindest Mikro-Hinterschneidungen bestehen und Wachs auch bei Raumtemperatur regelmässig einem Modellmaterial wie beispielsweise Gips gegenüber eine geringere Härte aufweist.

Aus der DE 196 42 247 ist ein Verfahren zur Herstellung von Zahnersatz bekannt, bei dem zunächst dreidimensionale Produktdaten erfasst und für die Herstellung des Zahnersatzes aufbereitet werden. Bei diesem Verfahren wird eine elektronisch gesteuerte Werkzeugmaschine eingesetzt, um eine schnelle Erzeugung eines Prototypen auf der Grundlage der Produktdaten zu gewährleisten. Zwar lässt sich mit diesem Verfahren der Zahnersatz recht genau herstellen, das Herstellverfahren ist jedoch recht aufwendig und bedingt eine elektronisch gesteuerte Werkzeugmaschine, die mittels eines Fräsverfahrens die gewünschte Bearbeitung vornimmt. Dieses Verfahren hat jedoch eine Reihe von Nachteilen, denn es entstehen Abfälle und Verunreinigungen, die in einer zahnärztlichen Praxis nicht tolerierbar sind. Darüber hinaus ist der so hergestellte Zahnersatz häufig noch mit den bekannten Verfahren zu beschichten, da das Ausgangsmaterial nicht die geforderte Ästhetik aufweist.

Ferner ist es an sich bekannt, eine dreidimensionale Drucktechnik für die schnelle Herstellung von Prototypen zu verwenden. Hierbei kommen zwei Verfahren zum Einsatz: Beim ersten Verfahren (3D-Printing), das vom Massachusetts Institute of Technology entwickelt wurde, wird ein pulverförmiges Material über eine Düsenanordnung schichtweise entsprechend dem herzustellenden Objekt mit einem Bindemittel versehen und das Bindemittel bindet schichtweise dabei das Pulver ab. Bei diesem Verfahren wird nach dem Fertigstellen des Objektes das überschüssige und ungebundene Pulver entfernt. Bei diesem Verfahren besteht eine freie Wahlmöglichkeit hinsichtlich des Pulvers, wobei jedoch regelmässig eine körnige Oberfläche verbleibt und Hohlräume, die die Festigkeit beeinflussen, sind nicht auszuschliessen. Nachbehandlungen des so erhaltenen Objektes zur Erhöhung der mechanischen Festigkeit und der Oberfläche sind hierbei in der Regel erforderlich.

Die WO 01/13841 A1 beschreibt ein solches Verfahren, bei dem über einen Druckkopf pulverförmiges Material sowie ein Bindemittel schichtweise aufgetragen wird. Auch aus der US-A-5,690,490 ist ein 3D-Printing-Verfahren bekannt, bei dem eine Matrix von Nadeln ein flüssiges Material schichtweise auf einen Grundkörper aufbringt. In beiden Fällen sind die erreichbaren Schichtdicken jedoch sehr gering.

Aus der US 5204124 ist ein 3D-Plottverfahren insbesondere zur Prototypenherstellung eines Bootskörpers bekannt, wobei das Kunststoffmaterial beim Auftragen kontinuierlich polymerisiert wird.

Bei einem weiteren Verfahren (Fused Deposition Modeling), das bspw. in der WO 01/15620 A1 beschrieben wird, wird mittels der dreidimensionalen Drucktechnik über elektrostatische Tintenstrahldüsen ein an Luft aushärtbares oder erstarrendes Material aufgebracht. Dieses Material wird aufgeschmolzen und über Düsen aufgetragen. Aufgrund der erforderlichen Durchhärtung ist jedes Materialteilchen recht klein, so dass die Herstellung eine entsprechend lange Zeit benötigt. Üblicherweise wird ein Materialstrang in einer 3D-dimensionierbaren Düse aufgeschmolzen und appliziert. Hierbei ist die Anwendung jedoch auf sehr wenige thermoplastische Materialien, z.B. ABS, beschränkt. Nachteil ist weiterhin, dass aus dem zu verarbeitenden Material erst ein Strang entsprechender Abmessung hergestellt werden muss, der anschliessend der Düsenanordnung zugeführt wird.

Weiterhin ist bekannt, computerunterstützt dreidimensionale Objekte herzustellen, wobei die dreidimensionale Struktur durch das schichtweise Einbringen von Mikropunkten oder Mikrosträngen in ein flüssiges Medium aufgebaut wird (Macromolecular Materials and Engineering 2000, 282, S. 17-21). Dadurch wird es möglich, Strukturen aus mechanisch instabilen Materialien aufzubauen, die durch die Kompensation der Schwerkraft durch den Auftrieb im flüssigen Medium ihre Geometrie nach dem Herstellen bis zu einer mechanischen oder chemischen Verfestigung beibehalten. Dieses Verfahren ermöglicht somit die Verwendung von Materialien, die bisher einem Rapid Prototyping nicht zugänglich waren. Die Anwendung dieses Verfahrens zur Herstellung von dentalen Produkten ist jedoch nicht beschrieben.

Daher liegt der Erfindung die Aufgabe zugrunde, ein Verfahren zur Herstellung von Dentalprodukten unter Verwendung des 3D-Plottings gemäss Anspruch 1 zu schaffen, das die Herstellung der verschiedenartig strukturierten Formteile aus den verschiedensten Materialien erlaubt und nur geringere Investitionskosten erfordert.

Diese Aufgabe wird erfindungsgemäss durch Anspruch 1 gelöst. Vorteilhafte Weiterbildungen ergeben sich aus den Unteransprüchen.

Mit dem erfindungsgemässen Verfahren lassen sich Dentalprodukte unter Verwendung des 3D-Plottings auf der Basis von schmelzbaren, kondensierbaren, thermisch oder mit UV- bzw. VIS-Licht härtbaren ungefüllten oder gefüllten Materialien mittels eines computergesteuerten 3D-Plotters herstellen. Mit diesem Verfahren wird es erstmals möglich, Dentalmaterialien auf der Basis hochviskoser bzw. gefüllter Ausgangsmaterialien durch computerunterstützte formfreie Fertigung herzustellen. Es erfolgt hierbei der Aufbau der 3D-Objekte schichtförmig durch eine computerkontrollierte Abscheidung von Mikrotropfen oder Mikrosträngen in einer Flüssigkeit oder auf einer festen Oberfläche. Je nach Art der verwendeten Materialien erfolgt die Härtung durch Abkühlung von geschmolzenen Materialien, durch chemische Reaktion oder eine Polymerisation wird thermisch bzw. durch gleichzeitige oder nachträgliche Bestrahlung ausgelöst. Darüber hinaus ist auch eine Aushärtung der Materialien durch eine chemische Reaktion mit einem flüssigen Medium möglich.

Das Ausbringen der Materialien erfolgt über eine pneumatische Einspeisung unter Verwendung von kommerziell verfügbaren Einweg-Dosierern mit einer Düsenöffnung von etwa 100 bis 2000 µm, wobei der Durchmesser der Düsenöffnungen und der Druck der treibenden Luft von der Viskosität des Materials abhängt. Weiterhin sind auch bei Zweikomponenten- Materialien Doppelkartuschen- Dosierer einsetzbar oder mehrere Kartuschen können im Verlauf des Druckens ausgetauscht werden. Während beim 3D-Printing nur dünnflüssige Stoffe bzw. Stoffgemische mit einem Bindemittel verarbeitet werden können, lassen sich mit dem 3D-Plotting hochviskose und gefüllte Stoffe und Stoffgemische verarbeiten, die bei wichtigen Dentalprodukten, z.B. künstlichen Zähnen, Füllungsmaterialien oder Verblend- oder Gerüstmaterialien, Verwendung finden. Zur Steuerung des 3D-Plotting-Prozesses können die z.B. beim Fräsen von Metallen bekannten CNC-Verfahren genutzt werden, wobei die Basisdaten über 3D-Scanner, 3D-Digitalfotografie oder auch aus dem medizinischen Bereich bekannten Abbildungsverfahren zugänglich sind.

Im Gegensatz zu den gängigen "Rapid Prototyping"-Verfahren lassen sich mit dem erfindungsgemässen Verfahren sehr verschiedenartig strukturierte Formteile erzeugen. Neben homogen aufgebauten Teilen sind so auch poröse Formteile oder auch Formteile, die aus unterschiedlichen Schichten nach einem Gradientenmuster bestehen, einfach herzustellen. Zudem können Gradienten durch Variation der Materialien im Verlaufe des Plottens erfolgen.

Dementsprechend lassen sich mit dem 3D-Plotting im Gegensatz zu den gängigen und bekannten "Rapid Prototyping"-Verfahren auf der Basis von Kompositen künstliche Zähne oder Inlays mit Schichtstruktur, glasfaserverstärkte Kronen- und Brückengerüste oder keramische Grünkörper für den dentalen Einsatz herstellen. Darüber hinaus ist das 3D-Plotting apparativ wesentlich weniger aufwendig als die bekannten Verfahren.

Durch die Verwendung des 3D-Plottings lassen sich zunächst gegenüber den bekannten Verfahren grössere Schichtstärken erzeugen, die durch das punkt- bzw. strangförmige Austragen der ungefüllten oder gefüllten Materialien entstehen. Dabei setzen sich die chemisch reaktiven Systeme aus mindestens zwei Komponenten zusammen, die unmittelbar vor dem Austragen innig vermischt werden und sofort nach dem Auftragen reagieren, also aushärten und eine formstabile Schicht bilden. Lichthärtbare Materialien enthalten in der Regel einen Photoinitiator. Nach dem Erzeugen dieser Schicht erfolgt eine Bestrahlung derselben mit Licht solcher Wellenlängen, mit denen der Photoinitiator aktiviert wird und die Aushärtung bewirkt. Im Gegensatz dazu verfügen keramische Systeme auf Grund ihrer Thixotropie über eine ausreichende Festigkeit, die ein Zwischenhärten der einzelnen Schichten überflüssig macht.

Dies erlaubt es, ein preisgünstiges Plotverfahren zu realisieren, wobei erfindungsgemäss eine Düse, die computergesteuert bewegt werden kann, einen Mikrostrang oder Mikrotropfen ausstossen kann. Der Durchmesser der Düse kann dabei durchaus bis 2 mm betragen, so dass ein rascher Schichtaufbau in der gewünschten Form realisierbar ist. Aufgrund der grossen Materialstärke wird ein rascher Aufbau des Dentalproduktes ermöglicht.

Erfindungsgemäss ist es besonders günstig, dass Teile für die Zahnrestauration bzw. für den Zahnersatz auf der Basis von schmelzbaren, kondensierbaren, thermisch oder mit Licht härtbaren ungefüllten oder verstärkten Materialien mittels eines computergesteuerten 3D-Plotters realisiert werden können. Besonders bevorzugt erfolgt der Aufbau der 3D-Objekte schichtförmig durch eine computergesteuerte Abscheidung von Mikrotropfen oder Mikrosträngen in einer Flüssigkeit oder alternativ trocken auf einer festen Oberfläche. Je nach Art der Materialien erfolgt die Härtung durch Abkühlung von geschmolzenen Materialien, durch Polykondensation bzw. Polyaddition oder wird eine Polymerisation thermisch oder durch gleichzeitige oder nachträglich Bestrahlung ausgelöst. Darüber hinaus ist die Aushärtung der Materialien durch chemische Reaktion mit einem flüssigen Medium möglich.

Es ist auch möglich, kommerziell verfügbare Einweg-Dosierer zu verwenden, die eine Düsenöffnung von 200 bis 2000 Mikrometer aufweisen können, wobei der Durchmesser der Düsenöffnung bzw. der auszuübende Druck für den Materialaustrag von der Viskosität des verwendeten Materials abhängt. Dabei versteht es sich von selbst, dass anstelle einer Düse auch mehrere Düsen zum Austragen verschiedener Materialien eingesetzt werden können.
Die Dosierung erfolgt bevorzugt über Druckluftbeaufschlagung des Einweg-Dosierers.

Alternativ kann auch ein Doppel- bzw. Mehrfachkartuschen-Dosierer eingesetzt werden, der es sogar ermöglicht, Materialien aus zwei Komponenten auszutragen, die beim Zusammenführen der Komponente in der Düse vermischt werden und nach dem Austragen aushärten.

Bei der Verwendung von lichthärtenden Materialien werden bekannte Lichtquellen eingesetzt, wobei es bevorzugt ist, auch UV-Anteile im Lichtspektrum vorzusehen.

Gemäss einem weiteren, besonders günstigen Aspekt der Erfindung lässt sich das erfindungsgemässe Verfahren gerade auch in den Zahnarztpraxen einsetzen.

Gemäss einer vorteilhaften Ausgestaltung der Erfindung ist es vorgesehen, den Schichtaufbau des herzustellenden Dentalproduktes an die natürliche Vorlage, z.B. einen Zahn, anzunähern. Das Zahnersatzteil weist häufig dem natürlichen Zahn angenähert Kunststoff- oder Keramikmaterial auf, das dem Zahnschmelz entspricht, aber auch Kunststoff- oder Keramikmaterial, das dem Dentin entspricht. Zusätzlich kommt bei Bedarf ein Opaker-Material zum Einsatz, beispielsweise zu Verblendungszwecken.

In vorteilhafter Ausgestaltung der Erfindung ist die Transluzenz der jeweils aufgebrachten Schicht an die geforderte Transluzenz des herzustellenden Zahnersatzteils angepasst. Soll beispielsweise die Labialseite eines Frontzahnes modelliert werde, erfolgt die Ausrichtung des Schichtaufbaus bevorzugt so, dass als letzte Schicht die labiale Abschlussschicht des Frontzahnersatzteils aufgebracht wird.

In besonders vorteilhafter Ausgestaltung der Erfindung werden solche Materialien oder Materialkombinationen verwendet, die eine bioaktive und/oder medizinische Wirkung besitzen, z.B. Fluorid-, Hydroxid- oder Calciumionen freisetzen. Darüber hinaus ist auch die Verwendung von Materialien möglich, die die Haftung von Plaque reduzieren oder auch eine gute Polierbarkeit zur Herstellung von ästhetischen Oberflächen aufweisen. Mit der Verwendung von keramischen Pasten können Grünkörper hergestellt werden, die nachträglich entsprechend dem Stand der Technik noch gehärtet werden.

Die nachfolgenden Beispiele verdeutlichen die Erfindung, erheben jedoch keinen Anspruch auf Vollständigkeit. Bei allen Beispielen wurde ein 3D-Plotter der Firma Envision Technologies verwendet. Der Düsendurchmesser der rechnergesteuerten Ploteinheit betrug für alle Beispiele 200 µm. Soweit nicht anders angegeben, wurden alle Beispiele in Luft ohne Verwendung eines reaktiven Mediums geplottet. Die Beispiele demonstrieren die Verwendung von nach dem Stand der Technik bekannten zahnärztlichen Restaurationsmaterialien für die Herstellung eines Inlays. Hierzu wurde eine Kavität in einem Zahn präpariert und die Kavität mit einem nach dem Stand der Technik bekannten Scannersystem vermessen. Mit den so gewonnenen Daten wurde anschliessend das Inlay wie nachfolgend beschrieben angefertigt.

### Beispiel 1:

### Anfertigung eines Inlays aus einem lichthärtenden, niedrigviskosen zahnärztlichen Füllungsmaterial

Zur Herstellung des Inlays wurde ein nach dem Stand der Technik bekanntes niedrigviskoses, lichthärtendes Füllungsmaterial verwendet (Tetric Flow der Firma Ivoclar-Vivadent AG). Zum Aufbau des Inlays wurden die Daten der Kavität in die rechnergesteuerte Ploteinheit transferiert. Der Aufbau des Inlays erfolgte durch computergesteuerte Abscheidung von Mikropunkten des verwendeten Materials auf einer Metalloberfläche. Die Aushärtung des verwendeten Materials erfolgte durch eine parallel zur Düse angebrachten, Licht im Wellenlängenbereich von 400 bis 500 nm emittierenden Lichtquelle. Der Aufbau der Kavität erfolgte derart, dass alle 10 Sekunden ein Mikropunkt geplottet wurde. In der Zeit zwischen dem Plotten zweier Punkte wurde jeweils die Austrittsöffnung der Düse abgedeckt und mit der oben genannten Lichtquelle ausgehärtet.

### Beispiel 2:

Beispiel 1 wurde unter Verwendung weiterer, nach dem Stand der Technik bekannten zahnärztlichen Füllungsmaterialien (Heliomolar Flow, Tetric Chroma und Ariston AT der Firma Ivoclar-Vivadent AG) wiederholt.

### Beispiel 3:

### Anfertigung eines Inlays aus einer lichthärtenden, kurzglasfasergefüllten Monomermischung

Zur Herstellung des Inlays wurde eine kurzglasfasergefüllte Monomermischung mit folgender Zusammensetzung verwendet. Die Angaben erfolgten hierbei in Gewichtsprozent.
30 % Kurzglasfasern (Hersteller Firma Schott) mittlere Länge 115 mm
29 % Bisphenol-A-Diglycidyldimethacrylat (Bis-GMA)
26 % 7,7,9-Trimethyl-4,13-dioxo-3,14-dioxa-5,12-diazahexadecan-1,16-dioxy-di-methacrylat
14 % Triethylenglykoldimethacrylat
1 % niedermolekulare Stabilisatoren, Additive und Initiatoren

Die Herstellung der kurzglasfasergefüllten Monomermischung erfolgte durch Einarbeitung der Kurzglasfasern in die Monomermischung mittels eines Rührwerks.
Mittels der rechnergesteuerten Ploteinheit wurde anschliesend das Inlay durch computergesteuerte Abscheidung von Mikrosträngen des verwendeten Materials auf einer Metalloberfläche aufgebaut. Die Aushärtung des verwendeten Materials erfolgte durch eine parallel zur Düse angebrachten, Licht im Wellenlängenbereich von 400 bis 500 nm emittierenden Lichtquelle. Hierzu wurde die Lichtquelle seitlich der aus lichtundurchlässigem Material angefertigten Düse angebracht.

### Beispiel 4:

### Anfertigung eines Inlays aus einem dualhärtenden (d.h. selbstund lichthärtend) zahnärztlichen Füllungsmaterial

Zur Herstellung des Inlays wurde ein nach dem Stand der Technik bekanntes dualhärtendes Füllungsmaterial verwendet (Variolink II Base und Cat der Firma Ivoclar-Vivadent AG). Zum Aufbau des Inlays wurden die Daten der Kavität in die rechnergesteuerte Ploteinheit transferiert. Der Aufbau des Inlays erfolgte durch computergesteuerte Abscheidung von Mikropunkten der verwendeten Materialien auf einer Metalloberfläche. Hierzu wurde eine Mischkartusche verwendet, diese erlaubt es, die zwei Komponenten Variolink II Base und Variolink II Cat erst unmittelbar vor dem Düsenaustritt zu gleichen Teilen miteinander zu mischen. Die Aushärtung des verwendeten Materials erfolgte selbsthärtend. Der Aufbau der Kavität erfolgte derart, dass alle 30 Sekunden ein Mikropunkt geplottet wurde.

### Beispiel 5:

Es wurde Beispiel 4 wiederholt unter der zusätzlichen Verwendung einer parallel zur Düse angebrachten, Licht im Wellenlängenbereich von 400 bis 500 nm emittierenden Lichtquelle. In der Zeit zwischen dem Plotten der Mikropunkte wurde die Düsenaustrittsöffnung abgedeckt.

### Beispiel 6:

Es wurde Beispiel 4 wiederholt unter Verwendung zweier Düsen. Jede dieser Düsen verfügte über einen separaten Vorratsbehälter. Einer dieser Behälter wurde mit Variolink II Base, der andere mit Variolink II Cat befüllt. Die Austritsöffnungen der beiden Düsen wurden derart ausgerichtet, das eine Vermischung der beiden Materialien erst nach dem Austreten aus der Düse stattfindet. Um eine ausreichende Zeitspanne für die Selbsthärtung des Materials zu gewährleisten, wurde der Zeitabstand zwischen dem Plotten zweier Punkte auf 60 Sekunden erhöht. Durch die Verwendung der in den vorigen Beispielen beschriebenen Lichtquelle an sich und deren Handhabung konnte diese Zeit auf 10 Sekunden verringert werden.

Als Polymermaterialien können eingesetzt werden reine Verbindungen oder Mischungen von polyreaktionsfähigen Monomeren, Oligomeren oder Polymeren, die vorzugsweise geeignete Füllstoffe und weitere Additive enthalten. Dabei kommen als Matrixsysteme vor allem in Frage:

### A. Polymerisierbare Matrixsysteme:

1. Radikalisch polymerisierbare Materialien auf der Basis von radikalisch polymerisierbaren Monomeren, wie (Meth)acrylate, Styrol und Styrolderivate, Allylverbindungen oder Vinylcyclopropane, wobei vor allem (Meth)acrylate besonders geeignet sind. Einsetzbar sind kommerziell verfügbare monofunktionelle Monomere, wie z.B. Methyl-, Ethyl-, Butyl-, Benzyl-, Furfuryl- oder Phenyl(meth)acrylat, und die als Vernetzermonomere bekannten mehrfunktionellen Acrylate bzw. Methacrylate wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglycoldi(meth)acrylat, Polyethylenglycoldimethacrylat, Trimethylolpropantri(meth)acrylat, Pentaerythrittetra(meth)acrylat sowie Butandioldi(meth)acrylat, 1,10-Decandioldi(meth)acrylat oder 1,12-Dodecandioldi(meth)acrylat.
2. Radikalisch polymerisierbare Oligomere oder Polymere, die endständige und/oder seitenständige radikalisch polymerisierbare Gruppen tragen, beispielsweise radikalisch polymerisierbare α, (5Mw(10U (meth)acryloyl-terminierte Polyester-, Polyether-, Polyepoxid-Amin- oder Polyurethan-Telechele oder Kieselsäurepolykondensate, die beispielsweise durch hydrolytische Kondensation von Silanen, die radikalisch polymerisierbare Gruppen, bevorzugt wie z.B. Methacryl- oder Acrylgruppen tragen, eingesetzt werden. Solche Kieselsäurepolykondensate sind beispielsweise in der DE-PS 4 416 857 oder der DE-PS 4 133 494 beschrieben.
   Die Aushärtung der radikalisch polymerisierbaren Stoffe erfolgt nach Zugabe geeigneter Initiatoren durch thermische, photochemische oder redoxinduzierte Polymerisation. Zur Initiierung der radikalischen Polymerisation werden vorzugsweise thermische und/oder Photoinitiatoren eingesetzt. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie weiter Azobisisobutyroethylester, Azobisisobutyronitril, Benzpinakol oder 2,2-Dimethylbenzpinakol. Beispiele für geeignete Photoinitiatoren sind Benzophenon, Benzoin sowie deren Derivate oder α-Diketone oder deren Derivate wie 9,10-Phenanthrenchinon, Diacetyl oder 4,4-Dichlorbenzil. Besonders bevorzugt werden Campherchinon und 2,2-Methoxy-2-phenyl-acetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethy-Ibenzoyldiphenyl- oder Bis(2,6-dichlorbenzoyl)-4-N-propylphenylphosphinoxid besonders geeignet.
   Als Initiatoren für eine bei Raumtemperatur durchgeführte Polymerisation werden Redox-Initiatorkombinationen, wie z.B. Kombinationen von Benzoyl- oder Laurylperoxid mit N,N-Dimethylsym.-xylidin oder N,N-Dimethyl-p-toluidin, verwendet.
3. Kationisch polymerisierbare Verdünner- oder Vernetzermonomere wie z.B. Glycidylether oder cycloaliphatische Epoxide, cyclische Ketenacetale, Vinylether, Spiroorthocarbonate, Oxetane oder bicyclische Orthoester. Beispiele sind: Triethylenglycoldivinylether, Cyclohexandimethanoldivinylether, 2-Methylen-1,4,6-trioxaspiro[2.2]-nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(-(3,4-epoxycyclohexylmethyl)adipat, Vinylcyclohexendioxid, 3-Ethyl-3-hydroxymethyloxetan, 1,10,-Decandiylbis(oxymethylen)bis(3-ethyloxetan) oder 3,3-(4-Xylylendioxy)-bis-(methyl-3-ethyloxetan). Als kationisch polymerisierbare Matrixsysteme eignen sich auch Kieselsäurepolykondensate, die beispielsweise durch hydrolytische Kondensation von Silanen, die kationisch polymerisierbare Gruppen, bevorzugt z.B. Epoxid-, Oxetan-, Spiroorthoester oder Vinylethergruppen tragen. Solche Kieselsäurepolykondensate sind beispielsweise in der DE-PS 4 133 494 oder US-PS 6 096 903 beschrieben.
   Für die Aushärtung von kationisch polymerisierbaren Systemen eignen sich besonders Diaryliodonium- oder Triarylsulfoniumsalze, wie z.B. Triphenylsulfoniumhexafluorophosphat oder -hexafluoroantimonat bzw. Systeme, die in der WO 97/13538 oder WO 98/47046 beschrieben sind.
4. Auch Materialien auf der Basis von Mischungen aus radikalisch und kationisch polymerisierbaren Verbindungen unter Verwendung entsprechender Initiatorkombinationen können eingesetzt werden, wobei die radikalische und kationische Polymerisation, gleichzeitig oder in aufeinander folgenden Stufen ablaufen.
5. Cyclische Monomere, die durch ringöffnende Metathesepolymerisation (RÖMP) vernetzen, wie monocyclische Alkene oder Alkadiene, beispielsweise Cyclopenten, Cyclohepten, Cycloocten, Cyclododecen oder 1,5-Cyclooctadien, oder bicyclische Alkene, beispielsweise Bicyclo[2.2.1]hept-2-en (2-Norbornen) bzw. davon ausgehende Derivate wie 7-Oxa-bicyclo[2.2.1]hept-2-en, Bicyclo[2.2.1]hept-5-en-2,3-dicarbon-säuredimethylester, 7-Oxabicyclo[2.2.1]hept-5-en-2,3-dicarbonsäurediethylester, 5-Norbornen-2-methylester bzw. 5-Norbornen-2-yl-ester von Mono-, Diund Multicarbonsäuren oder die Umsetzungsprodukte von 5-Norbornen-2-methanol bzw. 5-Norbornen-2-ol mit Mono- oder Diisocyanaten einsetzen. Dabei bilden solche Verbindungen, die mehrere Norbornengruppen enthalten, Polymernetzwerke. Für die Aushärtung lassen sich bekannte RÖMP-Katalysatoren, wie Metallcarben-Komplexe, z.B. Ru-, W- oder Mo-Carben-Komplexe (vgl. R. R. Schrock, Acc. Chem. Res. 23 (1990) 158), oder einfache Salze wie K₂RuCl₅ oder die Hydrate von RuCl₃ oder OsCl₃ bei polaren Monomeren (W. J. Feast, D. B. Harrison, Polymer 32 (1991) 558) einsetzen: Weiterhin eignen sich besonders luft- oder wasserstabile Katalysatoren auf dar Basis von Carbonylgruppen enthaltenden Mo-, Ru-, Os- oder W-Verbindun-gen mit einem Polyenliganden (WO 93/13171), z.B. [(C₆H₆)Ru(CH₃CN)₂CI]⁺PF₆⁻ oder als Photoinitiatoren photolabile Ru- oder Os-Verbindungen, wie z.B. [Os(C₆H₆)CI₂]₂ oder [Ru(C₆H₆)]₂(Tosylat)₂.

### B. Polyadditions-Matrixsysteme: Beispiele hierfür sind:

1. Polyurethan bzw. Polyharnstoff-Systeme: So sind bekanntlich Polyurethane aus Mischungen kommerziell zugänglicher Diisocyanate, wie z.B. Toluylendiisocyanat, Methylendiphenyldiisocyanat, 2,2,4- Trimethylhexamethylendiisocyanat oder Isophorondiisocyanat, bzw. daraus hergestellten oligomeren Polyisocyanaten mit geeigneten OH-multifunktionellen Verbindungen, wie Ethylenglycol, Glycerin oder Trimethylolpropan, bzw. daraus hergestellten di- oder trifunktionellen Polyolen zugänglich, wobei zinnorganische Verbindungen oder tertiäre Amine als Katalysator eingesetzt werden. Werden die Isocyanate mit Diaminen, wie z.B. Ethylendiamin, Hexamethylendiamin oder Bis(4-amino-3-methylcyclohexyl)methan, umgesetzt, so ergeben sich entsprechende Polyharnstoffe.
2. Epoxid-Harze: Polyaddukte von bekannten di- oder multifunktionellen Epoxidverbindungen, wie z.B. Tetrahydrophthalsäurediglycidylester, Bisphenol-A-diglycidylether, hydrierter Bisphenol-A-diglycidylether, Glycerintriglycidylether, 3,4-Epoxycyclohexylmethyl-3,4-epoxycyclohexancarboxylat oder Bis-(3,4-epoxy-cyclohexylmethyl)-adipat mit Di- oder Polyaminen, wie Ethylendiamin, Triethylentetramin, Diaminocyclohexan, Tricyclodecandiamin, Hexamethylendiamin oder m-Xylylendiamin bzw. Polyetherpolyaminen. Darüber hinaus kommen als Vernetzer auch Anhydride in Frage.
3. Thiol-En-Systeme: Umsetzungsprodukte von di- oder multifunktionelllen SH-Verbindungen, wie z.B. 1,4-Dimercaptobenzol, Trimethylolpropan-tris(3-mercaptopropionat) oder Pentaerythrit-tetrakis(3-mercaptopropionat) mit z.B. di-oder trifunktionellen Allyl- oder Norbornenverbindungen, wie z.B. Diallylether, Triallylisocyanurat oder Umsetzungsprodukte von 5-Norbornen-2-methanol bzw. 5-Norbornen-2-ol mit Diisocyanaten. Dabei kann die Thiol-En-Polyaddition durch bekannte radikalische Initiatoren, wie z.B. Azobisisobutyronitril, ausgelöst werden.
4. Michael-Reaktionsharze: Beispielsweise Umsetzungsprodukte von di- oder multifunktionellen Acrylaten mit di- oder multifunktionellen Acetoacetaten. Beispiele für geeignete Acrylate sind Ethylenglycoldiacrylat, Hexandioldiacrylat, Tripropylen-glycoldiacrylat, ethoxyliertes Bisphenol-A-Diacrylat, Polyethylenglycol-200-diacrylat, Trimethylolpropantriacrylat, Pentaerythrittetraacrylat. Diese Acrylate lassen sich insbesondere mit trioder tetrafunktionellen Acetoacetaten, wie z.B. Trimethylolpropan-und Glycerintrisacetoacetat sowie Pentaerythrittetrakis-acetoacetat zu Netzwerkpolymeren umsetzen. Als geeignete Katalysatoren werden vorzugsweise Alkalimetallhydroxide, wie z.B. KOH, Tetraalkylammoniumhydroxide, z.B. Tetrabutylammoniumhydroxid, insbesondere bicyclische Amidine, wie 1,5-Diazabicyclo[4.3.0]-5-nonen oder 1,8-Diazabicylo(5.4.0)-7-undecen, und Guanidine, vor allem Tetramethylguanidin eingesetzt.

### C) Polysiloxane

Schließlich kommen auch in Frage bekannte Silicon-Herze (vgl. W. Noll, Chemie und Technologie der Silicone, Verlag Chemie, Weinheim 1968; N. Auner, J. Weiss (Eds.),Organosilicon Chemistry, Wiley-VCH, 1997), die durch Kondensation oder Hydrosilylierung zu Polymernetzwerken führen.

Zur Herstellung von Kompositen werden den Matrixsystemen z. B. zur Verbesserung der mechanischen Eigenschaften organische oder anorganische Partikel oder Fasern zugegeben. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre oder mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengröße von 0,01 bis 5 Mikrometer sowie röntgenopake Füllstoffe, wie Ytterbiumtrifluorid. Darüber hinaus können auch kurze Glasfasern, Wiskers, Schichtsilikate, Polyamid- oder Kohlenstoff-Fasern eingesetzt werden.

Weiterhin können die Materialien für das 3D-Plotting weitere Additive enthalten, wie z.B. Farbmittel (Pigmente oder Farbstoffe), Stabilisatoren, Aromastoffe, mikrobiocide Wirkstoffe, Weichmacher oder UV-Absorber.

## Patentansprüche

1. Verfahren zur Herstellung eines dentalen Formteils, bei dem eine Unterlage bereitgestellt wird und auf diese ein polymerisierbarer Kunststoff für das Formteil schichtweise über eine rechnergesteuerte Auftragsvorrichtung in unpolymerisiertem Zustand mit dreidimensionaler Plottechnologie aufgetragen wird, wobei nach dem Auftragen einer Schicht diese ausgehärtet wird indem in der Zeit zwischen dem Plotten zweier Punkte die Austrittsöffnung der Düse der Auftragsvorrichtung abgedeckt wird und diese Punkte mit Licht polymerisiert werden, bevor das Auftragen der nächsten Schicht erfolgt.

2. Verfahren nach Anspruch 1, **dadurch gekennzeichnet, dass** die Polymerisation mittels UV-Licht, erfolgt und dass die Polymerisation von der Auftragvorrichtung beabstandet erfolgt.

3. Verfahren nach einem der vorhergehenden Ansprüche, **dadurch gekennzeichnet, dass** von der Auftragvorrichtung eine Vielzahl von Materialsträngen entsprechend der aufzubringenden Schicht aufgetragen werden.

4. Verfahren nach einem der vorhergehenden Ansprüche, **dadurch gekennzeichnet, dass** die Abscheidung des Materials durch die Auftragvorrichtung in eine Flüssigkeit erfolgt.

5. Verfahren nach einem der Ansprüche 1 bis 3, **dadurch gekennzeichnet, dass** das Auftragen durch die Auftragvorrichtung auf eine trockene Oberfläche erfolgt.

6. Verfahren nach einem der vorhergehenden Ansprüche, **dadurch gekennzeichnet, dass** jede Schicht nicht vollständig durchpolymerisiert, sondern soweit polymerisiert wird, dass sie eine ausreichende Festigkeit für das Auftragen der nächsten Schicht über ein dreidimensionales Auftragverfahren aufweist und dass sie in dem nächsten und den folgenden Schritten fertig polymerisiert wird.

7. Verfahren nach einen der vorhergehenden Ansprüche, **dadurch gekennzeichnet, dass** das Material ein polymerisierbares Dentalmaterial ist, das bis 70 Gewichtsprozent mindestens eines polymerisierbaren Monomers und/oder Oligomers, 0,1 bis 5 Gewichtsprozent mindestens eines Polymerisationsinitiators, bis 60 Gew.-% eines oder mehrerer Füllstoffe und mindestens 20 Gew.-% einer wachsartigen polymerisierbaren Substanz enthält.

8. Verfahren nach einem der vorhergehenden Ansprüche, **dadurch gekennzeichnet, dass** zusätzlich zur Polymerisation eine Polyaddition und/oder eine Polykondensation eingesetzt wird.

9. Verfahren nach einem der vorhergehenden Ansprüche, **dadurch gekennzeichnet, dass** die Auftragvorrichtung Einweg-Dosierer verwendet, insbesondere mit einer Düsenöffnung zwischen 200 und 2000 Mikrometer.

10. Verfahren nach einem der vorhergehenden Ansprüche, **dadurch gekennzeichnet, dass** nach der Lichthärtung eine thermische Nachbehandlung vorgenommen wird.

11. Verfahren nach einem der vorhergehenden Ansprüche, **dadurch gekennzeichnet, dass** polyreaktionsfähige Monomere, Oligomere oder Polymere als Material eingesetzt werden, die Füllstoffe enthalten und hochviskos sind.

12. Verfahren nach einem der vorhergehenden Ansprüche, **dadurch gekennzeichnet, dass** eine wachsartige polymerisierbare Substanz wie der Ester einer Carbonsäure mit einem polymerisationsfähigen Alkohol oder der Ester eines Alkohols mit einem polymerisationsfähigen Carbonsäurederivat mit einem Gewichtsanteil von etwa 1 bis 50% in dem Material enthalten ist.

13. Verfahren nach einem der vorhergehenden Ansprüche, **dadurch gekennzeichnet, dass** für die Herstellung des Formteils Material in unterschiedlichen Farben eingesetzt wird.

14. Verfahren nach einem der vorhergehenden Ansprüche, **dadurch gekennzeichnet, dass** die Schichtung aus Richtung des Kunststoffteils so gewählt ist, dass die oberste Schicht sich im wesentlichen parallel zur letzten aufzutragenden Schicht eines herzustellenden Zahns oder Zahnersatzteils, also der Zahnschmelz-Schicht, erstreckt.

15. Verfahren nach einem der vorhergehenden Ansprüche, **dadurch gekennzeichnet, dass** die Ausrichtung des Schichtaufbaus so gewählt ist, dass die maximale Länge und Breite des Kunststoffteils größer als seine Höhe ist.

16. Verfahren nach einem der vorhergehenden Ansprüche, **dadurch gekennzeichnet, dass** die oberste aufzubringende Schicht eine größere Transluzenz als darunterliegende Schichten aufweist.

17. Verfahren nach einem der vorhergehenden Ansprüche, **dadurch gekennzeichnet, dass** mindestens eine der Schichten einen pharmazeutischen Wirkstoff (z.B. Fluöridionen, einen Wirkstoff mit antibakteriellen Eigenschaften oder Ionen, die die Remineralisierung der Zahnhartsubstanz begünstigen bzw. fördern), freisetzt.

18. Verfahren nach einem der vorhergehenden Ansprüche **dadurch gekennzeichnet, dass** mindestens eine der Schichten Fasern zur Verstärkung der mechanischen Eigenschaften enthält.

19. Verfahren nach einem der vorhergehenden Ansprüche **dadurch gekennzeichnet, dass** mindestens eine der Schichten Säure neutralisierende Ionen freisetzt.

20. Verfahren nach einem der vorhergehenden Ansprüche **dadurch gekennzeichnet, dass** mindestens eine der Schichten opaliszierende Eigenschaften aufweist.

21. Verfahren nach einem der vorhergehenden Ansprüche **dadurch gekennzeichnet, dass** mindestens eine der Schichten auf Hochglanz polierbar ist.

22. Verfahren nach einem der vorhergehenden Ansprüche, **dadurch gekennzeichnet, dass** eine der Schichten geringe Plaqueadhäsion zeigt.

23. Verfahren nach einem der vorhergehenden Ansprüche, **dadurch gekennzeichnet, dass** mindestens eine der Schichten eine poröse Struktur aufweist.

24. Verfahren nach einem der vorhergehenden Ansprüche **dadurch gekennzeichnet, dass** bereits vorgefertigte dentale Formteile oder Halbzeuge mit einer Kunststoff- oder Keramikmasse beschichtet werden.

25. Verfahren nach Anspruch 24, **dadurch gekennzeichnet, dass** das vorgefertigte Formteil oder Halbzeug ein Implantat oder eine Prothese darstellt.

26. Verfahren nach Anspruch 24, **dadurch gekennzeichnet, dass** die Beschichtung bioaktive Eigenschaften aufweist.

27. Verfahren nach einem der vorhergehenden Ansprüche, **dadurch gekennzeichnet, dass** die Beschichtung eine harte oder weichbleibende Unterfütterung darstellt.

## Claims

1. A method for producing a dental form piece, said method providing a firm support onto which a polymerizable plastic material forming the form piece that has not yet polymerized is applied in a layer by layer manner with the aid of a computer-controlled material applying device by a three dimensional plotting technology, wherein a layer is cured after its application by covering the outlet of the nozzle of the material applying device during the time between the plotting of two spots and said spots being polymerized by light, before the next layer is applied.

2. A method for producing a dental form piece according to claim 1, wherein the polymerization is effected by means of UV light and wherein the polymerization takes place in a distance from the material applying device.

3. A method for producing a dental form piece according to one of the preceding claims, wherein the material applying device applies a plurality of material cords as a function of the layer to be applied.

4. A method for producing a dental form piece according to one of the preceding claims, wherein removing material is effected via the material applying device in a fluid medium.

5. A method for producing a dental form piece according to one of the claims 1 to 3, wherein the material applying device is controlled to apply the material on a dry surface.

6. A method for producing a dental form piece according to one of the preceding claims, wherein each of the applied layers is not completely polymerized, but to a condition in which the layer has a sufficient firmness in order to apply a next layer by a three-dimensional material applying technique and wherein the completion of the polymerization of the applied layer is accomplished during the next and subsequent steps.

7. A method for producing a dental form piece according to one of the preceding claims, wherein the plastic material is a polymerizable dental material having up to 70 percent by weight of at least one of a polymerizable monomer and/or a polymerizable oligomer, 0.1 to 5 percents by weight of at least one polymerization initiator, up to 60 percent by weight of one or more filling materials, and at least 20 percent by weight of a wax-like polymerizable substance.

8. A method for producing a dental form piece according to one of the preceding claims, wherein in additon to the polymerization process a polyaddition process and/or a polycondensation process are employed.

9. A method for producing a dental form piece according to one of the preceding claims, wherein the material applying device uses a one-way or disposable dosing device that in particular comprises a nozzle opening between 200 and 2000 micrometers.

10. A method for producing a dental form piece according to one of the preceding claims, wherein a thermal aftertreatment takes place subsequent to the light-curing process of the material applied.

11. A method for producing a dental form piece according to one of the preceding claims, wherein monomers, oligomers or polymers are used as plastic materials that are capable of performing a polyreaction and that contain filling materials and are very viscous.

12. A method for producing a dental form piece according to one of the preceding claims, wherein between about 1 to 50 percent in weight of the material applied is a wax-like polymerizable substance such as the ester of a carbon acid with a polymerizable alcohol or the ester of an alcohol with a polymerizable carbon acid derivative.

13. A method for producing a dental form piece according to one of the preceding claims, wherein material in different colours is used for producing the dental form piece.

14. Method for producing a dental form piece according to one of the preceding claims wherein the arrangement in layers in the direction of the plastic material is selected such that the outermost applied layer extends substantially parallel to the last layer to be applied of a tooth or tooth replacement part to be produced, that is to say parallel to the layer of the tooth enamel.

15. Method for producing a dental form piece according to one of the preceding claims, wherein the orientation of the layered structure is selected such that the maximum length and width of the plastic dental form piece is greater than its height.

16. Method for producing a dental form piece according to one of the preceding claims, wherein the outermost or top layer to be applied has a greater translucency than the applied layers arranged thereunder.

17. Method for producing a dental form piece according to one of the preceding claims, wherein at least one of the applied layers sets free a pharmaceutical active substance (e.g. fluoride ions, an active substance having anti-bacterial properties, or ions for assisting and promoting the remineralization of dental hard substances, that is to say tooth enamel, dentin and root cementum.

18. Method for producing a dental form piece according to one of the preceding claims, wherein at least one of the applied layers includes fibres for reinforcing or enhancing the mechanical properties of the applied material.

19. Method for producing a dental form piece according to one of the preceding claims, wherein at least one of the applied layers sets free acid-neutralizing ions.

20. Method for producing a dental form piece according to one of the preceding claims, wherein at least one of the layers has opalescence promoting properties.

21. Method for producing a dental form piece according to one of the preceding claims, wherein at least one of the layers is capable of being polished to mirror finish.

22. Method for producing a dental form piece according to one of the preceding claims, wherein one of the layers shows low plaque adhesion.

23. Method for producing a dental form piece according to one of the preceding claims, wherein at least one of the layers has a porous structure.

24. Method for producing a dental form piece according to one of the preceding claims, wherein prefabricated dental form pieces or semifinished dental form pieces are coated with a plastic or ceramic mass.

25. Method for producing a dental form piece according to claim 24, wherein the prefabricated or semifinished dental form piece is an implant or a prosthesis.

26. Method for producing a dental form piece according to claim 24, wherein the coating on the dental from piece has bioactive properties.

27. Method for producing a dental form piece according to one of the preceding claims, wherein the coating on the dental form piece provides a hard or soft-remaining lining.

## Revendications

1. Procédé de préparation d'une pièce dentaire, dans lequel une base est préparée et sur celle-ci, une matière plastique polymérisable pour la pièce est appliquée à l'état non polymérisé, par couches, à l'aide d'un dispositif d'application commandé par ordinateur avec une technologie de traçage tridimensionnelle,
où après l'application d'une couche, celle-ci est durcie avant l'application de la couche suivante, en ce que pendant la période entre le traçage de deux points, l'ouverture de sortie de la buse du dispositif d'application est couverte et ces points sont polymérisés avec de la lumière.

2. Procédé selon la revendication 1, **caractérisé en ce que** la polymérisation est réalisée à l'aide de lumière U.V. et **en ce que** la polymérisation est réalisée à distance du dispositif d'application.

3. Procédé selon l'une quelconque des revendications précédentes, **caractérisé en ce que** de multiples écheveaux de matériau correspondant à la couche à appliquer, sont appliqués par le dispositif d'application.

4. Procédé selon l'une quelconque des revendications précédentes, **caractérisé en ce que** le dépôt du matériau par le dispositif d'application est réalisé dans un liquide.

5. Procédé selon l'une quelconque des revendications 1 à 3, **caractérisé en ce que** l'application par le dispositif d'application est réalisée sur une surface sèche.

6. Procédé selon l'une quelconque des revendications précédentes, **caractérisé en ce que** chaque couche n'est pas complètement polymérisée, mais polymérisée suffisamment pour qu'elle présente une résistance suffisante pour l'application de la couche suivante par un procédé tridimensionnel d'application et **en ce qu'**elle est polymérisée complètement au cours de l'étape suivante et des étapes ultérieures.

7. Procédé selon l'une quelconque des revendications précédentes, **caractérisé en ce que** le matériau est un matériau dentaire polymérisable, qui contient jusqu'à 70% en poids d'au moins un monomère et/ou oligomère polymérisable, 0,1 à 5% en poids d'au moins un initiateur de polymérisation, jusqu'à 60% en poids d'une ou plusieurs charges et au moins 20% en poids d'une substance cireuse polymérisable.

8. Procédé selon l'une quelconque des revendications précédentes, **caractérisé en ce qu'**en plus de la polymérisation, une polyaddition et/ou une polycondensation sont mises en uvre.

9. Procédé selon l'une quelconque des revendications précédentes, **caractérisé en ce que** le dispositif d'application utilise un appareil de dosage à une voie, en particulier avec une ouverture de buse entre 200 et 2000 µm.

10. Procédé selon l'une quelconque des revendications précédentes, **caractérisé en ce qu'**après le durcissement par la lumière, on procède à un post-traitement thermique.

11. Procédé selon l'une quelconque des revendications précédentes, **caractérisé en ce que** les monomères, oligomères ou polymères polyréactionnels sont mis en £uvre en tant que matériau qui contient des charges et qui est très visqueux.

12. Procédé selon l'une quelconque des revendications précédentes, **caractérisé en ce qu'**une substance cireuse polymérisable, comme l'ester d'un acide carboxylique avec un alcool polymérisable, ou l'ester d'un alcool avec un acide carboxylique polymérisable, est présente en une proportion pondérale d'environ 1 à 50% dans le matériau.

13. Procédé selon l'une quelconque des revendications précédentes, **caractérisé en ce que** l'on met en £uvre un matériau de différentes teintes pour la préparation de la pièce.

14. Procédé selon l'une quelconque des revendications précédentes, **caractérisé en ce que** la disposition par couche dans le sens de la pièce plastique est choisie de sorte que la couche supérieure s'étende de manière essentiellement parallèle à la couche à appliquer en dernier d'une dent à fabriquer ou d'une pièce de prothèse dentaire, donc de la couche d'émail.

15. Procédé selon l'une quelconque des revendications précédentes, **caractérisé en ce que** l'organisation de la structure des couches est choisie de sorte que la longueur et la largeur maximales de la pièce plastique sont supérieures à sa hauteur.

16. Procédé selon l'une quelconque des revendications précédentes, **caractérisé en ce que** la couche supérieure à appliquer présente un caractère translucide plus élevé que les couches sous-jacentes.

17. Procédé selon l'une quelconque des revendications précédentes, **caractérisé en ce qu'**au moins une des couches libère une substance active pharmaceutique (par exemple, des ions fluorures, une substance active avec des propriétés antibactériennes ou des ions, qui favorisent ou accélèrent la reminéralisation des tissus durs de la dent).

18. Procédé selon l'une des revendications précédentes, **caractérisé en ce qu'**au moins une des couches contient des fibres pour le renforcement des propriétés mécaniques.

19. Procédé selon l'une des revendications précédentes, **caractérisé en ce qu'**au moins une des couches libère des ions neutralisant les acides.

20. Procédé selon l'une des revendications précédentes, **caractérisé en ce qu'**au moins une des couches présente des propriétés opalescentes.

21. Procédé selon l'une des revendications précédentes, **caractérisé en ce qu'**au moins une des couches peut être polie avec une brillance élevée.

22. Procédé selon l'une des revendications précédentes, **caractérisé en ce qu'**une des couches présente une faible adhésion de la plaque dentaire.

23. Procédé selon l'une des revendications précédentes, **caractérisé en ce qu'**au moins une des couches présente une structure poreuse.

24. Procédé selon l'une des revendications précédentes, **caractérisé en ce que** des pièces dentaires préalablement préparées ou des semi-finis sont revêtues d'une masse plastique ou céramique.

25. Procédé selon la revendication 24, **caractérisé en ce que** la pièce préalablement préparée ou le semi-fini est un implant ou une prothèse.

26. Procédé selon la revendication 24, **caractérisé en ce que** le revêtement présente des propriétés bioactives.

27. Procédé selon l'une des revendications précédentes, **caractérisé en ce que** le revêtement réalise une doublure dure ou restant blanche.
